(19) Europäisches Patentamt
European Patent Office
Office européen des brevets

(11) Publication number: **0 092 647**
**B1**

(12) # EUROPEAN PATENT SPECIFICATION

(45) Date of publication of patent specification: **14.01.87**

(21) Application number: **83100992.3**

(22) Date of filing: **03.02.83**

(51) Int. Cl.⁴: **C 07 D 307/52,**
C 07 D 405/12, A 61 K 31/34
// C07D295/12, C07D211/14,
C07D211/56, C07D203/12

(54) **New active compounds in the treatment of ulcers and skin allergy symptoms.**

(30) Priority: **27.04.82 IT 2095482**

(43) Date of publication of application:
**02.11.83 Bulletin 83/44**

(45) Publication of the grant of the patent:
**14.01.87 Bulletin 87/03**

(84) Designated Contracting States:
**AT BE CH DE FR GB LI LU NL SE**

(56) References cited:
**EP-A-0 002 930**

**The file contains technical information submitted after the application was filed and not included in this specification**

(73) Proprietor: **MAGIS FARMACEUTICI S.R.L.**
**Via Cacciamali n. 34/36/38 Zona Industriale - Loc. Noce**
**I-25100 Brescia (IT)**

(72) Inventor: **Moroni, Adolfo**
**Via Taramelli, 7**
**Brescia (IT)**

(74) Representative: **Gervasi, Gemma, Dr. et al**
**Studio Brevetti e Marchi NOTARBARTOLO & GERVASI 33, Viale Bianca Maria**
**I-20122 Milano (IT)**

**EP 0 092 647 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The object of this invention consists of new compounds which have anti-ulcerative action, the process for the preparation of these compounds and the pharmaceutical compositions that contain them.

More particularly, the object of this invention is concerned with the new compounds having formula I, shown below,

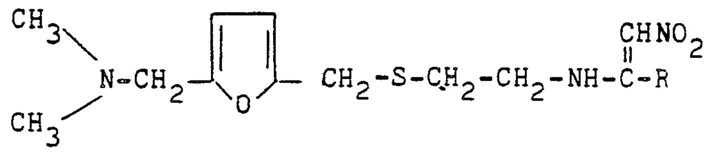

(I)

in which R is a piperidine group, 4-methylpiperidine, (3-N-ethyl-piperidinyl)amine, 4-(2-hydroxyethyl)-1-piperazine, 4-benzyl-piperidine, 4-benzyl-piperazine, ethyleneimine, propyleneimine, cyclohexylamine, 1.4-cyclohexadienylamine, hexamethyleneimine, (N-hexamethyleneimino)amine, cycloheptylamine, cyclopentylamine, pyrrolidine, morpholine, endo-2-norbornylamine, norbornen-2-yl-amine, and their pharmaceutically acceptable salts.

The pharmaceutically acceptable salts include, for example, non-toxic salts obtained by the addition of inorganic acids such as hydrochloric, bromic, iodic, phosphoric or sulphuric, and those obtained by the addition of organic acids such as maleic, acetic, citric, oxalic, succinic, benzoic, tartaric, fumaric, mandelic, ascorbic, methylsulphonic or ethylsulphonic.

EP—A—2930 discloses a very large number of compounds useful in the treatment of ulcers which can be represented by the formula (I) above wherein R represents a secondary amino group different from those defined above and comprising at least a carbocyclic or heterocyclic radical.

Another objective of this invention is a new process for the preparation of the compounds of formula (I), as defined above, and their pharmaceutically acceptable salts, characterized by the fact that the formula III compound shown below,

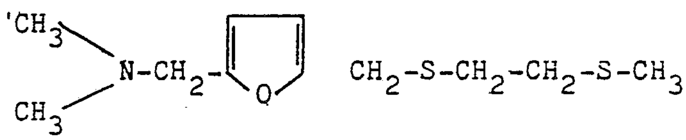

(III)

is made to react with the formula II compound, show below,

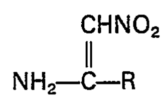

(II)

in which R is as previously defined, at a temperature of 80°C, after which the compound obtained is isolated and, optionally, treated to obtain the salts.

The formula I compounds, as defined above, and their pharmaceutically acceptable salts have shown themselves to be useful in the treatment of affections which require the administration of histamine —$H_2$ receivers as, for example, in cases of peptic ulcer or allergic sumptoms of the skin. Pharmaceutical compounds which contain the compounds of formula (I), as defined above, as principle ingredients and in effective amounts, or their pharmaceutically acceptable salts, just as they are or in combination with other compatible active ingredients and/or vehicles, diluents, solvents and/or pharmaceutically acceptable excipient, form a further objective of this invention.

Table A, shown below, shows the corresponding formula structure of R, together with the significances of R, as described above, and the code number assigned to each formula I compound which corresponds to each particular R structure, which will be used from here on for the sake of brevity.

TABLE A

| R Group | | Corresponding Formula I Compound Code Number |
|---|---|---|
| Structure Formula | Denomination | |
| 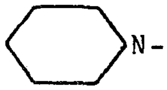 | piperidine | AU /001 |
| 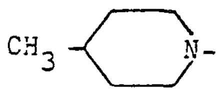 | 4-methyl-piperidine | AU /002 |
| 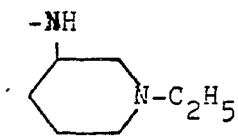 | (3-N-ethylpiperidinyl)amino | AU /003 |
| 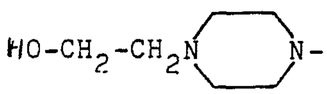 | 4-(2-hydroxyethyl)-1-piperazine | AU /004 |
| 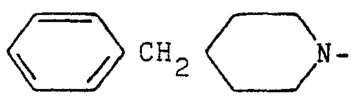 | 4-benzyl-piperidine | AU /005 |
| 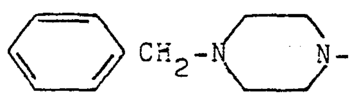 | 4-benzyl-1-piperazine | AU /006 |
| 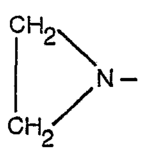 | ethyleneimine | AU /007 |
| 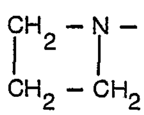 | propyleneimine | AU /008 |
| 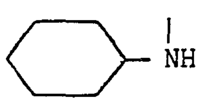 | cyclohexylamine | AU/009 |
| 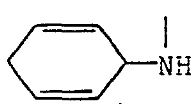 | 1.4-cyclohexadienylamine | AU /010 |
| 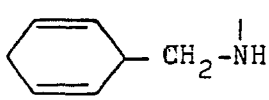 | 1.4-cyclohexadienyl-2-methylamine | AU/011 |

TABLE A (Continued)

| R Group<br>Structure Formula | Denomination | Corresponding Formula I Compound Code Number |
|---|---|---|
| 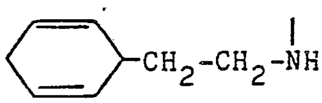 | 1.4-cyclohexadienyl-2-ethylamine | AU /012 |
| 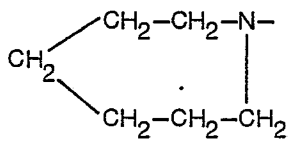 | hexamethyleneimine | AU /013 |
| 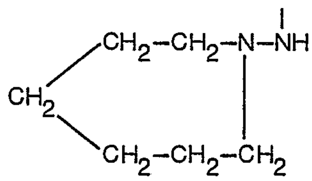 | N-aminohexamethyleneimine | AU /014 |
| 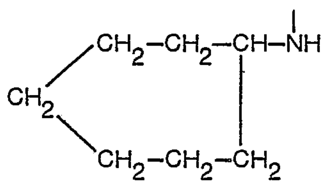 | cycloheptylamine | AU /015 |
| 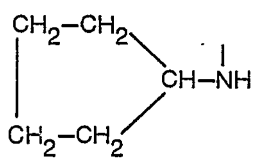 | cyclopentylamine | AU /016 |
| 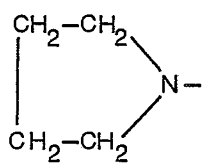 | pyrrolidine | AU /017 |
| 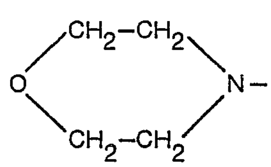 | morpholine | AU /018 |
| 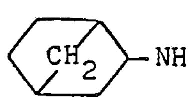 | endo-2-norbornylamine | AU /019 |
| 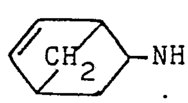 | norbornen-2-yl-amine | AU /020 |

The formula (I) compounds and their pharmaceutically acceptable salts are active in the treatment of affections which require the administration of histamine —$H_2$ receivers, such as, for example, peptic ulcer and allergic symptoms of the skin. For example, they present anti-ulcer activity which is superior to that provided by Ranitidine and by cimetidine, active principles that are already well known in the field. Specifically, they are 1.1 and 1.3 times superior to Ranitidine and from 5 to 10 times superior to cimetidine. Furthermore, the formula I compounds and their pharmaceutically acceptable salts present practically complete absence of any collateral effects. These characteristics of elevated activity and practical absence of collateral effects have been evaluated by studying the acute toxicity, subchronic toxicity, chronic toxicity, foetal toxicity and cardiocirculatory effects on ulcers with pylorus ligature and of containment, on gastric secretion and on tetrafastrin gastric hyperactivity. The low toxicity value and the absence of collateral effects afford optimum value of therapeutic index, better than that of prior art compounds as those described in EP—A—2930. Acute toxicity was studied in male and female Swiss mice and in male and female, albino Wistar rats, administering the active principle orally, by venal injection and by muscular injection.

Each type of administration was carried out with geometrically progressive doses on 10 animals for each dose over an observation period of 10 days. At the end of the observation period, the $DL_{50}$ and relative confidence limits using the Litchfield and Wilcoxon method (Pharmacol Exp. Ther., 96—19, 1949) were calculated.

The results are shown in the following table, Table No. 1.

TABLE 1

| DL50 (Fid. Lim. 95) mg/kg | Administration in Mouse | | | Administration in Rat | |
|---|---|---|---|---|---|
| | orally | in vein | in muscle | in vein | in muscle |
| AU/001 | 2600 | 90 | 300 | 100 | 2100 |
| AU/002 | 2700 | 100 | 320 | 100 | 2000 |
| AU/003 | 2800 | 90 | 300 | 100 | 2200 |
| AU/004 | 2700 | 95 | 300 | 110 | 2000 |
| AU/005 | 2700 | 95 | 300 | 110 | 2100 |
| AU/007 | 2800 | 100 | 310 | 100 | 2100 |
| AU/008 | 2600 | 100 | 310 | 100 | 2200 |
| AU/009 | 2600 | 100 | 300 | 110 | 2200 |
| AU/010 | 2700 | 95 | 310 | 110 | 2200 |
| AU/011 | 2700 | 95 | 310 | 110 | 2100 |
| AU/012 | 2800 | 90 | 300 | 110 | 2100 |
| AU/013 | 2700 | 90 | 300 | 100 | 2100 |
| AU/014 | 2600 | 90 | 300 | 100 | 2200 |
| AU/015 | 2800 | 100 | 300 | 100 | 2200 |
| AU/016 | 2700 | 90 | 300 | 100 | 2200 |
| AU/017 | 2600 | 100 | 300 | 100 | 2200 |
| AU/018 | 2700 | 90 | 350 | 100 | 2200 |
| AU/019 | 2600 | 100 | 300 | 120 | 2100 |
| AU/020 | 2700 | 110 | 300 | 110 | 2100 |

The subchronic toxicity test, for each compound examined, was carried out on 100 albino rats (Sprague-Dawley strain, 50 males and 50 females) weighing on the average of about 120 grams each.

The rats were kept under standard environmental conditions with standard diets. They were divided into four groups with 25 animals in each group. Each group of animals was given the dose shown in Table 2, below, by injection into the vein, once a day, every day of the week for a total period of 4 weeks.

TABLE 2

| | | |
|---|---|---|
| Group I: | control group (physiological solution) | |
| Group II: | AU/001-002-003-004-005-006-007-008-009-010-011-012-013-014-015-016-017-018-019-020 | (1 mg/kg) |
| Group III: | AU/001-002-003-004-005-006-007-008-009-010-011-012-013-014-015-016-017-018-019-020 | (5 mg/kg) |
| Group IV: | AU/001-002-003-004-005-006-007-008-009-010-011-012-013-014-015-016-017-018-019-020 | (10 mg/kg) |

The test was also carried out for each product examined on 10 adult Beagle hounds, 5 male and 5 females, each weighing about 10 kg.

The dogs were kept under standard environmental conditions with standard diets. They were divided into two groups with 5 dogs in each group. Each dog was administered a dose once a day, every day of the week for a total period of 4 weeks, with Group I being the control group and receiving a physiological solution and Group II receiving the AU/001-002-003-004-005-006-007-008-009-010-011-012-013-014-015-016-017-018-019-020 compounds in doses of 5 mg/kg,

The condition of the rats and dogs, both of the control groups and the ones receiving the products under examination, always remained at a good level.

All the haemotological, haemochemical, urine and haemopathological tests gave results which were within the standard limits. None of the tests revealed any variations that were attributable to the treatment being carried out with the doses being administered as stated above.

The chronic toxicity test was conducted for each compound being examined on 60 albino rats, males and females, of the Sprague-Dawley strain, with an average weight of 97±5 grams, and on male and female Beagle hounds, with an average weight of 10 kg.

The treatment was carried out by oral administration for both the dogs and the rats (gastric tube for the rats), seven times a week for a period of 24 weeks, with equal doses of all 20 compounds, AU/001 to AU/020, as described in Table A. These doses are listed in the following table, Table No. 3.

TABLE 3

| Animal | Group | Method of Administration | Dose of Preferred Compounds |
|---|---|---|---|
| rat | I | gastric tube | vehicle |
| rat | II | gastric tube | 25 mg/kg |
| rat | III | gastric tube | 50 mg/kg |
| rat | IV | gastric tube | 100 mg/kg |
| dog | I | daily diet | no treatment |
| dog | II | daily diet | 25 mg/kg |
| dog | III | daily diet | 50 mg/kg |
| dog | IV | daily diet | 100 mg/kg |

The administration of the daily doses orally of the compounds examined did not provoke any modification whatsoever of the haematological, haemochemical and urinary constants of the animals nor did they cause any changes in the macro and microscopic aspects of the principle organs.

No changes were noted as regards their body weight and there were no mortalities.

The administered doses, at any rate, were greater than those foreseen for human therapy.

The foetal toxicity test was carried out on 40 male and 60 female Sprague-Dawley albino rats, weighing an average of 125 grams each.

Both the males and the females were administered 0,25, 50 and 100 mg/kg doses, of each of the compounds being examined, orally. The groups of 10 males each were administered the doses for a period of 60 days preceding the mating and the groups of 20 females each were administered the doses for a period of 15 days preceding the mating.

The tests were also carried out for all of the compounds being examined by administration to 40 adult, New Zealand White rabbits, weighing an average of 3 kg. The doses were of 0, 20, 40 and 60 mg/kg and administered orally between the 6th and 18th days of pregnancy. The foetal toxicity tests produced results which assure that there is absolutely no negative interference on the pregnancy process and the conception products.

The administered doses, in any case were greater than what is foreseen for human therapy.

All the compounds listed in Table A, from AU/001 to AU/020, were examined.

The test for evaluating the cardiocirculatory effects was carried out, for each of the compounds being examined, on 6 male, New Zealand White rabbits, averaging 2.5 kg in weight each. They were anesthetized with ethyl urethane and three of the animals received 100 mg/kg doses orally and the other three animals received 20 mg/kg doses intravenously. No changes were noted in the arterial pressure, the depth and frequency of breathing nor in the electrocardiograms. All the compounds listed in Table A, from AU/001 to AU/020, were examined.

Study of the effects of the new compounds of this invention were carried out using 50 male and female, Sprague-Dawley rats, weighing about 200 grams each, for each compound examined. The animals were made to fast and then underwent ligature of the pylorus.

One hour following the intervention, the animals were given oral doses of either 1, 3, 10 and 30 mg/kg of the compounds being examined, 3, 10, 30 and 100 mg/kg of Cimetidine or 1, 3, 10 and 30 mg/kg of Ranitidine.

The administration of the compounds of this invention, as well as the Cimetidine and Ranitidine, notably curbed the degree of gastric ulcer induced by the typing off the pylorus.

The activity level of the compounds of this invention is about 3 times greater than the Cimetidine and from 1.2 to 1.3 times greater than the Ranitidine. All the compounds listed in Table A, from AU/001 to AU/020, were examined.

For the study of the effects of the new compounds of this invention on containment ulcers, 60 Sprague-Dawley, albino, male rats, weighing an average of 200 grams each, were used. One group of the animals received a vehicle only and served as a control group.

Two other groups were given a pretreatment of 10 and 20 mg/kg doses of the compounds under examination, the doses given orally.

Two other groups, before being placed in the containment cages, were given 10 and 20 mg/kg oral doses of Cimetidine. The final two remaining groups of animals received 3 and 10 mg/kg oral doses of Ranitidine before being put into the containment cages.

The incidence of gastric ulcer, provoked experimentally through cold containment, is reduced by the administration of the compounds of this invention to a greater extent than that observed with doses of Cimetidine, which are 3.3 times greater, and with doses of Ranitidine, which are 1.1 and 1.2 times greater. All the compounds in Table A, from AU/001 to AU/020, were examined.

From the study of the curative effects of the new compounds of this invention on containment ulcers, 30 Sprague-Dawley, male, albino rats, weighing an average of 200 grams each, were used for testing each of the products under examination.

The animals were made to fast for 16 hours and then placed in containment cages, at a temperature of 21°C, for a period of 24 hours.

They were then freed and treated for a maximum period of 15 days with the products in accordance with the following program:

Group I: control group animals sacrificed after 5 days (5 animals) and after 15 days (5 animals)

Group II: compounds examined: 2.5 mg/kg orally; sacrificed after 5 days (5 animals) and after 15 days (5 animals)

Group III: compounds examined: 5 mg/kg orally; sacrificed after 5 days (5 animals) and after 15 days (5 animals).

The repeated administration of the examined compounds favors the healing of the containment ulcers. All the compounds in Table A, from AU/001 to AU/020, were examined.

The new compounds of this invention, coded AU/001 to AU/020 in Table A, which were administered both orally and intravenously in doses, respectively, of 0, 25, 0 and 50 mg/kg and of 0, 10, 0 and 20 mg/kg to Sprague-Dawley, male, albino rats, significantly reduces the volume of gastric secretion and gastric acidity without provoking any pH change of the gastric juices.

For the study of the effect of the new compounds of this invention on gastric hyperactivity induced by

tetragastrin, 30 Sprague-Dawley, male albino rats, weighing an average of 180 grams each, were used for each of the compounds being examined.

The animals were not fed for 16 hours and then kept anesthetized with ethyl urethane. A tube was inserted into the stomachs of the animals for obtaining the gastric secretions, according to the Ishii and Shinoraki, Jap. J. Pharmacol. 18,93 — 1968 technique, after having tied off the pylorus and the cervical region of the oesophagus.

After having prepared the animals in the manner described, tetragastrin was administered subcutaneously in 0.5 mg/kg doses. At the same time, the animals received physiological solution treatment (control) and intravenous injections of the compounds examined in doses of 0.25 mg/kg and 0.50 mg/kg.

The hyperacidity induced by the administration of the tetragastrin proved to be significantly inhibited by the intravenous treatment with the compounds examined. All the compounds in Table A, from AU/001 to AU/020, were examined.

Thanks to their characteristics of higher activity and practical absence of collateral effects, the compounds of formula (I) and their pharmaceutically acceptable salts are particularly useful as active principles in the formulation of pharmaceutical compositions. They, therefore, constitute another objective part of this invention, since these pharmaceutical compositions are characterized by the fact that the active principle that they contain consists of an effective quantity of one or more formula (I) compounds or their pharmaceutically acceptable salts, per se, or combined with compatible active principles and/or vehicles, diluents, solvents and/or excipients which are pharmaceutically acceptable.

These pharmaceutical compositions can be formulated so as to be administered orally, rectally, by injection or locally. They can be in solid form as, for example, capsules, pills, retarded-action pills, single-dose envelopes, suppositories, ointment and salves, as well as liquid form, such as solutions, suspensions and emulsions, which can be used directly or prepared extemporaneously. All the above-mentioned pharmaceutical compositions can be formulated to contain diluents, vehicles, solvents and/or excipients that are well known in the field and can be prepared according to the methods well known in the field and amply described in, for example, "Technologia Farmaceutica", Silvano Casadio — Ed. Cisalpino Goliardica — Milano, 1972.

The new compounds which are object of this invention can be administered just as they are or in the form of pharmaceutically acceptable salts, in daily quantities of from 0.2 to 50 mg/kg or, preferably, from 0.5 to 20 mg/kg, taken in partial doses two to four times a day in posological units containing, for example 10, 20, 30, 50, 100, 200, 250 or 500 mg/kg of active principle.

Another fact that constitutes an objective of this invention is that the compounds of formula (I) and their pharmaceutically acceptable salts can be made by reacting 2[[[5 - [(dimethylamino)methyl] - 2 - furanyl]methyl]thio]ethylmethylsulphide, which has the following formula (III).

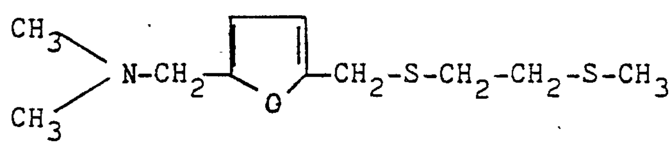

(III)

with the compound in formula (II) below,

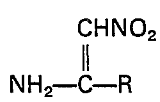

in which R is as defined above, at a temperature of 80°C, then isolating the obtained formula (I) compound and, optionally, producing the salts.

An excess of the formula (II) compound proves advantageous and the reaction takes place in three hours.

Another fact which constitutes an objective of this invention is that the formula (II) compound can be obtained by reacting 1,1-bis-(methylthio)-2-nitroethylene, which has the formula (IV) below,

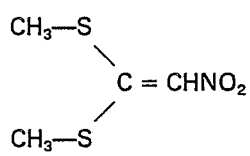

with a compound having formula (V) below,

$$RH \qquad (V)$$

in which R is as defined above, and then treating with ammonia.

This reaction can be usefully carried out in organic solvents and, advantageously, with tetrachloroethane.

The resulting product can be purified by passing it through a column of silica gel and successive crystallization.

The formula (I) compounds can be easily isolated using the methodology that is well known as, for example, by crystallization from solvents. These solvents are appropriately made of mixtures of water and ethyl alcohol. The optional production of the salts can also be done in accordance with well-known techniques as, for example, the adding of the appropriate acid.

The following examples show certain kinds of teacting pertinent to this invention, but these do not in any way limit the invention.

Example 1

1 - N - [2 - [[5 - [(dimethylamino)methyl]furfuryl]thio]ethyl]amino - 1 - piperidino - 2 - nitro ethylene (AU/001). 24.54 grams of 2[[5 - [(dimethylamino)methyl]furfuryl]thio]ethylmethylsulphide are made to react with 35 grams of 1-amino-1-piperidino-2-nitro ethylene at 80°C for 3 hours. The mixture is allowed to cool and restabilized with ethyl alcohol. The mixture is filtered and the precipitate is dissolved in ethyl alcohol. It precipitates again as water. A mixture of water and ethyl alcohol is used to crystallize the product. The product melts at between 101 and 105°C.

Spectrophotometric analyses confirm the structure.

Elemental analysis $C_{17}H_{28}N_4O_3S$ Molecular weight 368.481.

| | | | | |
|---|---|---|---|---|
| Calculated: | C 55,51%; | H 7.66%, | N 15.21%, | S 8.70% |
| Found: | C 55.80%; | H 7.50%; | N 15.20%; | S 8.60% |

Example 2

1 - N - [2 - [[5 - [(dimethylamino)methyl]thio]ethyl]amino - 1 - (4 - methyl - piperidino) - 2 - nitro ethylene (AU/002).

24.54 grams of 2 - [[5[(dimethylamino)methyl]furfuryl]thio]ethylmethylsulphide are made to react with 37 grams of 1-amino-1-(4-methyl-piperidino)-2-nitro ethylene at 80°C for three hours.

The mixture is allowed to cool and the process continues as described for Example No. 1.

The product melts at from 105 to 110°C. Spectrophotometric analyses confirm the structure.

Elemental analysis $C_{18}H_{30}N_4O_3S$ Molecular weight 382.508.

| | | | | |
|---|---|---|---|---|
| Calculated: | C 56.52%; | H 7.91%, | N 14.65%, | S 8.38% |
| Found: | C 57.00%; | H 7.90%; | N 15.00%; | S 8.35% |

Example 3

N - [2 - [[5 - [(dimethylamino)methyl]furfuryl]thio]ethyl] - N' - (N - ethylpiperidinyl) - 2 - nitro - 1.1 - ethylenediamine (AU/003).

24.54 grams of 2 - [[5 - [(dimethylamino)methyl]furfuryl]thio]ethylmethylsulphide are made to react with 40 grams of 2-nitro-N-ethylpiperidinyl)-1.1-ethylenediamine at 80°C for three hours.

The mixture is allowed to cool and the process continues as described for Example No. 1.

The product melts at between 96 and 98°C. Spectrophotometric analyses confirm the structure.

Elemental analysis $C_{19}H_{32}N_5SO_3$ Molecular weight 410.57.

| | | | | |
|---|---|---|---|---|
| Calculated: | C 55.58%; | H 7.86%, | N 17.06%, | S 7.81% |
| Found: | C 55.60%; | H 7.85%; | N 17.10%; | S 7.82% |

Example 4

1 - N - [2 - [[5 - [(dimethylamino)methyl]furfuryl]thio]ethyl] - amino - 1 - [4 - (2 - hydroxyethyl) - 1 - piperazine]-2-nitro ethylene (AU/004).

24.54 grams of 2 - [[5 - [(dimethylamino)methyl]thio]ethylmethylsulphide are made to react with 43 grams of 1-amino-1-[4-(2-hydroxyethyl)-piperazino]-2-nitro ethylene at 80°C for three hours. The mixture is allowed to cool and the process continues as described for Example No. 1.

The product melts at between 95 and 101°C. Spectrophotometric analyses confirm the structure.

Elemental analysis $C_{18}H_{31}N_5SO_4$ Molecular weight 413.55.

| | | | | |
|---|---|---|---|---|
| Calculated: | C 52.28%; | H 7.56%, | N 16.92%, | S 7.75% |
| Found: | C 52.30%; | H 7.60%; | N 17.00%; | S 7.80% |

Example 5

1 - N - [2 - [[5 - [(dimethylamino)methyl]furfuryl]thio]ethyl] - amino - 1 - (4 - benzyl - piperidino) - 2 - nitro ethylene (AU/005).

24.54 grams of 2 - [[5 - [(dimethylamino)methyl]furfuryl]thio]ethylmethylsulphide are made to react with 52 grams of 1-amino-1-(4-benzyl-piperidine)-2-nitro ethylene at 80°C for three hours.

The mixture is allowed to cool and the process continued as described for Example 1.

The product melts at between 102 and 106°C. Spectrophotometric analyses confirm the structure.

Elemental analysis $C_{24}H_{34}N_4SO_3$ Molecular weight 458.64.

| | | | | |
|---|---|---|---|---|
| Calculated: | C 62.85%; | H 7.47%, | N 12.22%, | S 6.99% |
| Found: | C 63.00%; | H 7.50%; | N 12.30%; | S 7.00% |

Example 6

1 - N - [2 - [[5 - [(dimethylamino)methyl]furfuryl]thio]ethyl] - amino - 1 - (4 - benzyl - piperidino) - 2 - nitro ethylene (AU/006).

24.54 grams of 2 - [[5 - [(dimethylamino)methyl]furfuryl]thio]ethylmethylsulphide are made to react with 53 grams of 1-amino-1-(4-benzyl-1-piperidine)-2-nitro ethylene at 80°C for three hours.

The mixture is allowed to cool and the process continues as described for Example No. 1.

The product melts at between 104 and 108°C. Spectrophotoletric analyses confirm the structure.

Elemental analysis $C_{23}H_{33}N_5SO_3$ Molecular weight 459.61.

Calculated: C 60.11%; H 7.24%, N 15.24%, S 6.98%
Found: C 60.00%; H 7.20%; N 13.30%; S 7.00%

Example 7

1 - N - [2 - [[5 - [(dimethylamino)methyl]furfuryl]thio]ethyl] - amino - 1 - ethyleneimino - 2 - nitro ethylene (AU/007).

24.54 grams of 2 - [[5 - [(dimethylamino)methyl]furfuryl]thio]ethylmethylsulphide are made to react with 26 grams of 1-amino-1-ethyleneimine-2-nitro ethylene at 80°C for three hours.

The mixture is allowed to cool and the process continues as described for Example 1.

The product melts at between 107 and 110°C. Spectrophotometric analyses confirm the structure.

Elemental analysis $C_{14}H_{22}N_4SO_3$ Molecular weight 326.46.

Calculated: C 51.51%; H 6.79%, N 17.16%, S 9.82%
Found: C 51.60%; H 6.80%; N 17.20%; S 9.80%

Example 8

1 - N - [2 - [[5 - [(dimethylamino)methyl]furfuryl]thio]ethyl] - amino - 1 - cyclopropylenimine - 2 - nitro ethylene (AU/008).

24.54 grams of 2 - [[5 - [(dimethylamino)methyl]furfuryl]thio]ethylmethylsulphide are made to react with 31 grams of 1-amino-1-propyleneimino-2-nitro ethylene at 80°C for three hours.

The mixture is allowed to cool and the process continues as described for Example 1.

The product melts at between 106 and 109°C. Spectrophotometric analyses confirm the structure.

Elemental analysis $C_{15}H_{24}N_4SO_3$ Molecular weight 340.46.

Calculated: C 52.33%; H 7.11%, N 16.46%, S 9.42%
Found: C 52.30%; H 7.20%; N 16.50%; S 9.45%

Example 9

N - [2 - [[5 - [(dimethylamino)methyl]furfuryl]thio]ethyl] - N' - (cyclohexyl) - 2 - nitro - 1.1 - ethylenediamine (AU/009).

24.54 grams of 2 - [[5 - [(dimethylamino)methyl]furfuryl]thio]ethylmethylsulphide are made to react with 37 grams of 2-nitro-N-cyclohexyl-1.1-ethylenediamine at 80°C for three hours.

The mixture is allowed to cool and the process continues as described for Example 1.

The product melts at between 97 and 99°C. Spectrophotometric analyses confirm the structure.

Elemental analysis $C_{18}H_{30}N_4SO_3$ Molecular weight 382.53.

Calculated: C 56.52%; H 7.91%, N 14.65%, S 8.38%
Found: C 56.60%; H 8.00%; N 14.70%; S 8.40%

Example 10

N - [2 - [[5 - [(dimethylamino)methyl]furfuryl]thio]ethyl] - N' - (1.4 - cyclohexadienyl) - 2 - nitro - 1.1 - ethylenediamine (AU/010).

24.54 grams of 2 - [[5 - [(dimethylamino)methyl]furfuryl]-2-thio]ethylmethylsulphide are made to react with 36 grams of 2-nitro-N-(1.4-cyclohexadienyl)-1.1-ethylenediamine at 80°C for 3 hours.

The mixture is allowed to cool and the process continues as described for Example No. 1.

The product melts at between 103 and 107°C. Spectrophotometric analyses confirms the structure.

Elemental analysis $C_{18}H_{28}N_4SO_4$ Molecular weight 380.53.

Calculated: C 56.82%; H 7.42%, N 14.72%, S 8.43%
Found: C 57.00%; H 7.45%; N 15.00%; S 8.40%

Example 11

N - [2 - [[5 - [(dimethylamino)methyl]furfuryl]thio]ethyl] - N' - (1.4 - cyclohexadienyl - 2 - methyl) - 2 - nitro - 1.1 - ethylenediamine (AU/011).

24.54 grams of 2 - [[5 - [(dimethylamino)methyl]furfuryl]thio]ethylmethylsulphide are made to react with 40 grams of 2-nitro-N'-(1.4-cyclohexadienyl-2-methyl)-1.1-ethylenediamine at 80°C for 3 hours.

The mixture is allowed to cool and the process continues as described for Example No. 1.

The product melts at between 99 and 103°C. Spectrophotometric analyses confirms the structure.

Elemental analysis $C_{19}H_{28}N_4SO_4$ Molecular weight 386.52.

Calculated: C 59.04%; H 5.75%, N 14.50%, S 8.29%
Found: C 60.00%; H 5.80%; N 14.40%; S 8.30%

Example 12

N - [2 - [[5 - [(dimethylamino)methyl]furfuryl]thio]ethyl] - N' - (1.4 - cyclohexadienyl - 2 - ethyl) - 2 - nitro - 1.1 - ethylenediamine (AU/012).

24.54 grams of 2 - [[5 - [(dimethylamino)methyl]furfuryl]thio]ethylmethylsulphide are made to react with 41 grams of 2-nitro-N'-(1.4-cyclohexadienyl-2-ethyl)-1.1-ethylenediamine at 80°C for 3 hours.

The mixture is allowed to cool and the process continues as described for Example No. 1.

The analyses made spectrophotometrically confirm the structure of the obtained compound.

Elemental analysis $C_{20}H_{30}N_4SO_3$ Molecular weight 406.55.

Calculated: C 59.08%; H 7.44%, N 13.78%, S 7.89%
Found: C 60.00%; H 7.50%; N 13.70%; S 7.90%

Example 13

1 - N - [2 - [[5 - [(dimethylamino)methyl]furfuryl]thio]ethyl] - amino - 1 - hexamethyleneimino - 2 - nitro - ethylene (AU/013).

24.54 grams of 2 - [[5 - [(dimethylamino)methyl]furfuryl]thio]ethylmethylsulphide are made to react with 37 grams of 1-amino-1-hexamethyleneimino)-ethylene at 80°C for three hours.

The mixture is allowed to cool and the process continues as described for Example No. 1.

The analyses made spectrophotometrically confirm the structure of the obtained compound.

Elemental analysis $C_{18}H_{30}N_4SO_3$ Molecular weight 382.56.

Calculated: C 56.51%; H 7.90%, N 14.65%, S 8.38%
Found: C 56.80%; H 8.00%; N 14.60%; S 8.40%

Example 14

N - [2 - [[5 - [(dimethylamino)methyl]furfuryl]thio]ethyl] - N' - (N - hexamethyleneimino)amine - 2 - nitro - 1.1 - ethylenediamine (AU/014).

24.54 grams of 2 - [[5 - [(dimethylamino)methyl]furfuryl]thio]ethylmethylsulphide are made to react with 40 grams of 2-nitro-N-hexamethyleneimino-1.1-ethylenediamine at 80°C for three hours.

The mixture is allowed to cool and the process continues as described for Example No. 1.

The analyses made spectrophotometrically confirm the structure of the obtained compound.

Elemental analysis $C_{18}H_{31}N_5SO_3$ Molecular weight 397.57.

Calculated: C 54.38%; H 7.86%, N 17.62%, S 8.06%
Found: C 55.00%; H 7.90%; N 17.70%; S 8.00%

Example 15

N - [2 - [[5 - [(dimethylamino)methyl]furfuryl]thio]ethyl] - N' - cycloheptyl - 2 - nitro - 1.1 - ethylenediamine (AU/015).

24.54 grams of 2 - [[5 - [(dimethylamino)methyl]furfuryl]thio]ethylmethylsulphide are made to react with 40 grams of 2-nitro-N-cycloheptyl-1.1-ethylenediamine at 80°C for three hours.

The mixture is allowed to cool and the process continues as described for Example No. 1.

The product melts at between 91 and 94°C. Spectrophotometric analyses confirms the structure.

Elemental analysis $C_{19}H_{33}N_4SO_3$ Molecular weight 397.56.

Calculated: C 57.40%; H 8.37%, N 14.09%, S 8.06%
Found: C 57.50%; H 8.40%; N 14.10%; S 8.10%

Example 16

N - [2 - [[5 - [(dimethylamino)methyl]furfuryl]thio]ethyl] - N' - cyclopentyl - 2 - nitro - 1.1 - ethylenediamine (AU/016).

24.54 grams of 2 - [[5 - [(dimethylamino)methyl]furfuryl]thio]ethylmethylsulphide are made to react with 42 grams of 2-nitro-N-cyclopentyl-1.1-ethylenediamine at 80°C for three hours.

The mixture is allowed to cool and the process continues as described for Example No. 1.

The product melts at between 97 and 103°C. Spectrophotometric analyses confirms the structure.

Elemental analysis $C_{17}H_{28}N_4SO_3$ Molecular weight 369.46.

Calculated: C 55.27%; H 7.64%, N 15.17%, S 8.68%
Found: C 55.30%; H 7.60%; N 15.20%; S 8.70%

Example 17

1 - N - [2 - [[5 - [(dimethylamino)methyl]furfuryl]thio]ethyl] - amino - 1 - pyrrolidine - 2 - nitro - ethylene (AU/017).

24.54 grams of 2 - [[5 - [(dimethylamino)methyl]furfuryl]thio]ethylmethylsulphide are made to react with 33 grams of 1-amino-1-pyrrolidino-2-nitro ethylene at 80°C for three hours.

The mixture is allowed to cool and the process continues as described for Example No. 1.

The product melts at between 95 and 100°C. Spectrophotometric analyses confirms the structure.

Elemental analysis $C_{16}H_{26}N_4SO_4$ Molecular weight 354.48.

Calculated: C 54.20%; H 7.39%, N 15.81%, S 9.04%
Found: C 54.10%; H 7.40%; N 15.80%; S 9.10%

Example 18

1 - N - [2 - [[5 - [(dimethylamino)methyl]furfuryl]thio]ethyl] - amino - 1 - morpholine - 2 - nitro - ethylene (AU/018).

24.54 grams of 2 - [[5 - [(dimethylamino)methyl]furfuryl]thio]ethylmethylsulphide are made to react with 35 grams of 1-amino-1-morpholine-2-nitro ethylene at 80°C for three hours.

The mixture is allowed to cool and the process continues as described for Example No. 1.

The product melts at between 98 and 103°C. Spectrophotometric analyses confirms the structure.

Elemental analysis $C_{16}H_{26}N_4SO_4$ Molecular weight 370.45.

| | | | | |
|---|---|---|---|---|
| Calculated: | C 51.88%; | H 7.08%, | N 15.12%, | S 8.65% |
| Found: | C 52.00%; | H 7.10%; | N 15.20%; | S 8.60% |

Example 19

N - [2 - [[5 - [(dimethylamino)methyl]furfuryl]thio]ethyl] - N' - (endo - 2 - norbornyl) - 2 - nitro - 1.1 - ethylenediamine (AU/019).

24.54 grams of 2 - [[5 - [(dimethylamino)methyl]furfuryl]thio]ethylmethylsulphide are made to react with 40 grams of 2-nitro-N'-(endo-2-norbornyl)-1.1-ethylenediamine at 80°C for three hours.

The mixture is allowed to cool and the process continues as described for Example No. 1.

Spectrophotometric analyses confirms the structure of the obtained compound. The product melts at between 95 and 100°C.

Elemental analysis $C_{19}H_{30}N_4SO_3$ Molecular weight 394.54.

| | | | | |
|---|---|---|---|---|
| Calculated: | C 57.84%; | H 7.66%, | N 14.28%, | S 8.13% |
| Found: | C 57.90%; | H 7.65%; | N 14.30%; | S 8.10% |

Example 20

N - [2 - [[5 - [(dimethylamino)methyl]furfuryl]thio]ethyl] - N' - (norbornen - 2 - yl) - 2 - nitro - 1.1 - ethylenediamine (AU/020).

24.54 grams of 2 - [[5 - [(dimethylamino)methyl]furfuryl]thio]ethylmethylsulphide are made to react with 40 grams of 2-nitro-N-(norbornen-2-yl)-1.1-endodiamine at 80°C for three hours.

The mixture is allowed to cool and the process continues as described for Example No. 1.

The product melts at between 95 and 101°C. Spectrophotometric analyses confirms the structure of the obtained product.

Elemental analysis $C_{19}H_{28}N_4SO_3$ Molecular weight 392.53.

| | | | | |
|---|---|---|---|---|
| Calculated: | C 58.14%; | H 7.18%, | N 14.28%, | S 8.17% |
| Found: | C 58.20%; | H 7.20%; | N 14.30%; | S 8.12% |

Example 21

1-amino-1-piperidinyl-2-nitro-ethylene.

16.5 grams of 1.1-bis-(methylthio)-2-nitro ethylene are dissolved in 100 ml of heated tetrachloroethane. 8.5 grams of piperidine are added. The solution is heated under movement for a period of two hours. The solution is then allowed to cool and anhydrous ammonia gas is added until saturation occurs.

The solution is agitated for two hours. The solvent is evaporated with the help of a vacuum and the product obtained is purified through a chromatographic column of silica gel. The solution is eluted by means of petroleum ether, first, and then with dichloroethane. The separating product crystallizes from diethyl ether and melts at between 121 and 123°C.

Example 22

1-amino-1-(4-methyl-piperidinyl)-2-nitro ethylene.

The process is used as in Example No. 21, except that 9.9 grams of 4-methyl-piperidine are used in place of the piperidine.

The product obtained melts at between 125 and 128°C.

Example 23

N-(3-N-ethylpiperidinyl)-2-nitro-ethylenamine.

The same process is used as in Example No. 21, except that 16.6 grams of 3-amino-N-ethylpiperidine are used in place of the piperidine.

The product obtained melts at between 118 and 120°C.

Example 24

1-amino-1-[4-(2-hydroxyethyl)-piperazinyl]-2-nitro ethylene.

The same process is used as in Example No. 21, using equimolecular quantities of 1.1-bis-(methylthio)-2-nitro ethylene and 4-(2-hydroxyethyl)-1-piperazine.

The product obtained melts at between 125 and 128°C.

Example 25

1-amino-1-(4-benzyl piperidinyl)-2-nitro ethylene.

The same process is used as in Example No. 21, using equimolecular quantities of 1.1-bis-(methylthio)-2-nitro ethylene and 4-benzyl-piperidine and saturating with ammonia.

The product obtained melts at between 125 and 128°C.

Example 26

1-amino-1-(4-benzyl-1-piperazinyl)-2-nitro ethylene.

Example No. 21 process, equimolecular amounts of 1.1-bis-(methylthio)-2-nitro ethylene and 4-benzyl-1-piperazine, saturating with ammonia. The product obtained melts at between 118 and 121°C.

Example 27

1-amino-1-ethyleneimine-2-nitro ethylene.

The same process is used as in Example No. 21, using equimolecular quantities of 1.1-bis-(methylthio)-2-nitro ethylene and ethylenamine followed by saturation with ammonia.

The product obtained melts at between 98 and 100°C.

Example 28

1-amino-1-propyleneimine-2-nitro ethylene.

The same process is used as in Example No. 21, using equimolecular quantities of 1.1-bis-(methylthio)-2-nitro ethylene and propyleneimine followed by saturation with ammonia.

The product obtained melts at between 111 and 115°C.

Example 29

N-cyclohexyl-2-nitro-1.1-ethylenediamine.

The same process is used as in Example No. 21, using equimolecular quantities of 1.1-bis-(methylthio)-2-nitro ethylene and cyclohexylamine followed by saturation with ammonia.

The product obtained melts at between 115 and 118°C.

Example 30

N-(1.4-cyclohexadienyl)-2-nitro-1.1-ethylenediamine.

The same process is used as in Example No. 2, using equimolecular quantities; of 1.1-bis-(methylthio)-2-nitro ethylene and 1.4-cyclohexadienylamine followed by saturation with ammonia.

The product obtained melts at between 120 and 125°C.

Example 31

N-(1.4-cyclohexadienyl-2-methyl)-2-nitro-1.1-ethylenediamine.

The same process is used as in Example No. 21, using equimolecular quantities of 1.1-bis-(methylthio)-2-nitro ethylene and 1.4-cyclohexadienyl-2-methylamine followed by saturation with ammonia.

The product obtained melts at between 125 and 128°C.

Example 32

N-(1.4-cyclohexadienyl-2-ethyl)-2-nitro-1.1-ethylenediamine.

The same process is used as in Example No. 21, using equimolecular quantities of 1.1-bis-(methylthio)-2-nitro ethylene and 1.4-cyclohexadienyl-2-ethylamine followed by saturation with ammonia.

The product obtained melts at between 120 and 125°C.

Example 33

1-amino-1-hexamethyleneimine-2-nitro ethylene.

The same process is used as in Example No. 21, using equimolecular quantities of 1.1-bis-(methylthio)-2-nitro ethylene and hexamethyleneimine followed by saturation with ammonia.

Example 34

N-cycloheptyl-2-nitro-1.1-ethylenediamine.

The same process is used as in Example No. 21, using equimolecular quantities of 1.1-bis-(methylthio)-2-nitro ethylene and cycloheptylamine followed by saturation with ammonia.

The product obtained melts at between 118 and 125°C.

Example 35

N-cyclopentyl-2-nitro-1.1-ethylenediamine.

The same process is used as in Example No. 21, using equimolecular quantities of 1.1-bis-(methylthio)-2-nitro ethylene and cyclopentylamine followed by saturation with ammonia.

The product obtained melts at between 117 and 124°C.

Example 36

1-amino-1-pyrrolidinyl-2-nitro ethylene.

The same process is used as in Example No. 21, using equimolecular quantities of 1.1-bis-(methylthio)-2-nitro ethylene and pyrrolidine followed by saturation with ammonia.

The product obtained melts at between 120 and 125°C.

Example 37

1-amino-1-morpholinyl-2-nitro ethylene.

The same process is used as in Example No. 21, using equimolecular quantities of 1.1-bis-(methylthio)-2-nitro ethylene and morpholine followed by saturation with ammonia.

The product obtained melts at between 120 and 125°C.

Example 38

N-(endo-2-norbornyl)-2-nitro-1.1-ethylenediamine.

The same process is used as in Example No. 21, using equimolecular quantities of 1.1-bis-(methylthio)-2-nitro ethylene and endo-2-norbornylamine followed by saturation with ammonia.

The product obtained melts at between 130 and 131°C.

Example 39

N-(norbornen-2-yl)-2-nitro-1.1-ethylenediamine.

The same process is used as in Example No. 21, using equimolecular quantities of 1.1-bis-(methylthio)-2-nitro ethylene and norbornen-2-yl-amine followed by saturation with ammonia.

The product obtained melts at between 128 and 132°C.

Example 40

N-hexamethyleneimine-2-nitro-1.1-ethylenediamine.

The same process is used as in Example 21, using equimolecular quantities of 1.1-bis-(methylthio)-2-nitro ethylene and N-amino-hexamethyleneimine followed by saturation with ammonia.

The product obtained melts at between 127 and 131°C.

**Claims for the Contracting States: BE CH DE FR GB LI LU NL SE**

1. Formula (I) compounds:

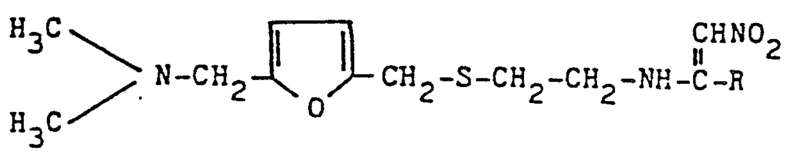

(I)

in which R is a piperidine group, 4-methylpiperidine, (3-N-ethyl-piperidinyl)amine, 4-(2-hydroxyethyl)-1-piperazine, 4-benzyl-piperidine, 4-benzyl-piperazine, ethyleneimine, propyleneimine, cyclohexylamine, 1.4-cyclohexadienylamine, hexamethyleneimine, (N-hexamethyleneimine)amine, cycloheptylamine, cyclopentylamine, pyrrolidine, morpholine, endo-2-norbornylamine, norbornen-2-yl-amine and their pharmaceutically acceptable salts.

2. Compound as per claim 1, characterized in that the said pharmaceutically acceptable salt is the salt obtained from the acids hydrochloric, hydrobromic, hydroiodic, phosphoric, sulphuric, maleic, acetic, citric, oxalic, succinic, benzoic, tartaric, fumaric, mandelic, ascorbic, methylsulphonic or ethylsulphonic.

3. Compound as per claim 1, characterized in that it is 1-N-[2-[[5-[dimethylamino)methyl]furfuryl]-thio]ethyl]-amino-1-piperidinyl-2-nitro ethylene.

4. Compound as per claim 1, characterized in that it is 1-N-[2-[[5-[dimethylamino)methyl]furfuryl]-thio]ethyl]-amino-1-(4-methyl-piperidinyl-2-nitro ethylene.

5. Compound as per claim 1, characterized in that it is 1-N-[2-[[5-[dimethylamino)methyl]furfuryl]-thio]ethyl]-N'-(N-ethylpiperidinyl)-2-nitro-1.1-ethylenediamine.

6. Compound as per claim 1, characterized in that it is 1-N-[2-[[5-[dimethylamino)methyl]furfuryl]-thio]ethyl]-amine-[4-(2-hydroxyethyl)-1-piperazinyl]-2-nitro ethylene.

7. Compound as per claim 1, characterized by the fact that it is 1-N-[2-[[5-[dimethylamino)methyl]-furfuryl]thio]ethyl]-amine-1-(4-benzyl-piperidinyl)-2-nitro ethylene.

8. Compound as per claim 1, characterized in that it is 1-N-[2-[[5-[dimethylamino)methyl]furfuryl]-thio]ethyl]-amine-1-(4-benzyl-piperazinyl)-2-nitro ethylene.

9. Compound as per claim 1, characterized in that it is 1-N-[2-[[5-[dimethylamino)methyl]-furfuryl]thio]ethyl]-amine-1-ethyleneimino)-2-nitro ethylene.

10. Compound as per claim 1, characterized by the fact that it is 1-N-[2-[[5-[dimethylamino)methyl]-furfuryl]thio]ethyl]-amine-1-propyleneimino-2-nitro ethylene.

11. Compound as per claim 1, characterized in that it is 1-N-[2-[[5-[dimethylamino)methyl]-furfuryl]thio]ethyl]-N'-cyclohexyl-2-nitro-1.1-ethylenediamine.

12. Compound as per claim 1, characterized in that it is 1-N-[2-[[5-[dimethylamino)methyl]-furfuryl]thio]ethyl]-N'-(1.4-cyclohexadienyl)-2-nitro-1.1-ethylenediamine.

13. Compound as per claim 1, characterized in that it is 1-N-[2-[[5-[dimethylamino)methyl]-furfuryl]thio]ethyl]-amine-1-hexamethylenenimino-2-nitro ethylene.

14. Compound as per claim 1, characterized in that it is N-[2-[[5-[dimethylamino)methyl]-furfuryl]thio]ethyl]-N'-(N-hexamethyleneimino)amino-2-nitro-1.1-ethylenediamine.

15. Compound as per claim 1, characterized in that it is N-[2-[[5-[dimethylamino)methyl]-furfuryl]thio]ethyl]-N'-cycloheptyl-2-nitro-1.1-ethylenediamine.

16. Compound as per claim 1, characterized in that it is N-[2-[[5-[dimethylamino)methyl]-furfuryl]thio]ethyl]-N'-cyclopentyl-2-nitro-1.1-ethylenediamine.

17. Compound as per claim 1, characterized in that it is 1-N-[2-[[5-[dimethylamino)methyl]-furfuryl]thio]ethyl]-amine-1-pyrrolidinyl-2-nitro ethylene.

18. Compound as per claim 1, characterized in that it is 1-N-[2-[[5-[dimethylamino)methyl]-furfuryl]thio]ethyl]-amine-1-morpholinyl-2-nitro ethylene.

19. Compound as per claim 1, characterized in that it is N-[2-[[5-[dimethylamino)methyl]-furfuryl]thio]ethyl]-N'-(endo-norbornyl)-2-nitro-1.1-ethylenediamine.

20. Compound as per claim 1, characterized in that it is N-[2-[[5-[dimethylamino)methyl]-furfuryl]thio]ethyl]-N'-(norbornen-2-yl)-2-nitro-1.1-ethylenediamine.

21. Compound characterized in that it is the pharmaceutically acceptable salt of a compound as per one of the claims 3 to 20.

22. Procedure for the preparing of formula (I) compounds, as defined in claim 1, and their pharmaceutically acceptable salts, characterized in that 2[[5-[dimethylamino)methyl]furfuryl]thio]ethylmethylsulphide, with formula (III) below,

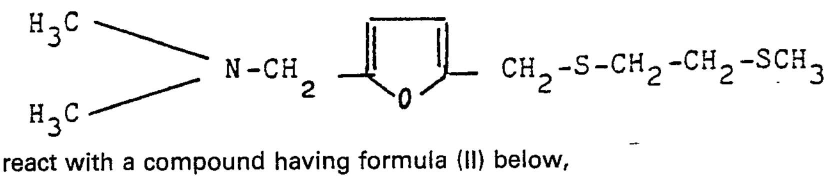

(III)

is made to react with a compound having formula (II) below,

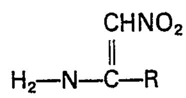

(II)

in which R is as defined in claim 1, at a temperature of 80°C whereafter the product obtained is isolated and, optionally converted to the desired salt.

23. Procedure as per claim 22, characterized in that an excess of formula (II) compound is used and the reaction mixture is held at 80°C for three hours.

24. Procedure as per one of the claims 22 and 23, characterized in that formula (II) compound is obtained by the reaction between 1.1-bis-(methylthio)-2-nitro-ethylene, the formula of which is (IV) below,

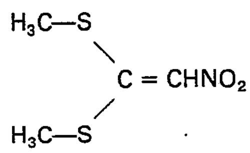

(IV)

and a compound having the formula (V) below,

RH (V)

in which R is as defined in claim 1, followed by reaction with ammonia.

25. Pharmaceutical composition characterized in that it contains, as active principle, an effective quantity of one or more of the compounds as per claims 1 to 21, 'per se' or combined with other compatible active principles, and/or vehicles, diluents, solvent and or excipients which are pharmaceutically acceptable.

26. Compositions as per claim 25, characterized in that it is suitable for administration orally, by injection, rectally or topically.

27. Composition as per claim 26, characterized in that it is in pill form.

28. Composition as per claim 26, characterized in that it is in the form of a pill having a prolonged effect with the active principle being released in small amounts over a period of time.

**Claims for the Contracting State: AT**

1. Process for the preparation of compounds of formula

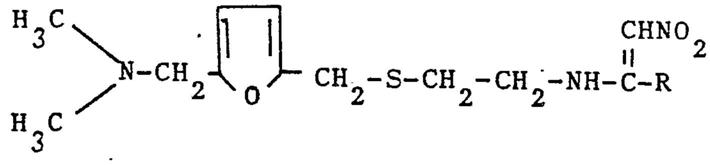

(I)

in which R is a piperidine group, 4-methylpiperidine, (3-N-ethyl-piperidinyl)amine, 4-(2-hydroxyethyl)-1-piperazine, 4-benzyl-piperidine, 4-benzyl-piperazine, ethyleneimine, propyleneimine, cyclohexylamine, 1.4-cyclohexadienylamine, hexamethyleneimine, (N-hexamethyleneimino)amine, cycloheptylamine, cyclopentylamine, pyrrolidine, morpholine, endo-2-norbornylamine, norbornen-2-yl-amine and their pharmaceutically acceptable salts, characterized in that 2-[[5-(dimethylamino)methyl]furfuryl]thio ethylmethylsulphide of formula

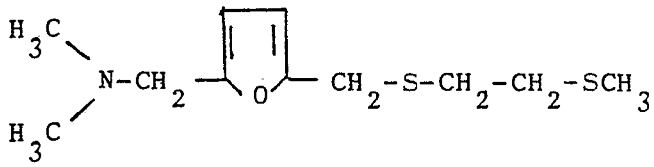

(III)

is reacted with a compound having the formula

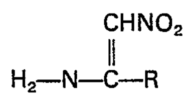

(II)

in which R is as above defined at a temperature of 80°C, whereafter the product obtained is isolated and, optionally converted to the desired salt.

2. Process according to claim 1, characterized in that an excess of formula (II) compound is used and the reaction mixture is held at 80°C for three hours.

3. Process according to claims 1 and 2, characterized in that formula (II) compound is obtained by the reaction between 1.1-bis-(methylthio)-2-nitro-ethylene of formula

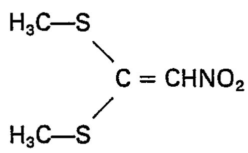

(IV)

and a compound having the formula

RH (V)

in which R is as defined in claim 1, followed by reaction with ammonia.

**Patentansprüche fur die Vertragstaaten: BE CH DE FR GB LI LU NL und SE**

1. Verbindungen der Formel (I)

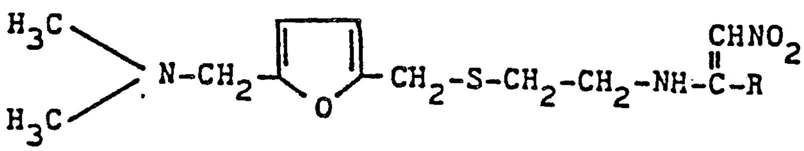

(I),

worin R eine Piperidinogruppe, 4-Methylpiperidin, (3-N-Äthylpiperidinyl)-amin, 4-(2-Hydroxyäthyl)-1-piperazin, 4-Benzylpiperidin, 4-Benzylpiperazin, Äthylenimin, Propylenimin, Cyclohexylamin, 1,4-Cyclohexadienylamin, Hexamethylenimin, (N-Hexamethylenimin)-amin, Cycloheptylamin, Cyclopentylamin, Pyrrolidin, Morpholin, Endo-2-norbornylamin, Norbornen-2-ylamin, ist, und deren pharmazeutisch annehmbare Salze.

2. Verbindung nach Anspruch 1, dadurch gekennzeichnet, daß des pharmazeutisch annehmbare Salz das Salz ist, das von Salz-, Bromwasserstoff-, Jodwasserstoff-, Phosphor-, Schwefel-, Malein-, Essig-, Citronen-, Oxal-, Bernstein-, Benzoe-, Wein, Fumar, Mandel-, Ascorbin-, Methylsulfon-, oder Äthylsulfonsäure erhalten wird.

3. Verbindung nach Anspruch 1, dadurch gekennzeichnet, daß sie 1-N-[2-[[5-[(Dimethylamino)-methyl]-furfuryl]-thio]-äthyl]-amino-1-piperidinyl-2-nitroäthylen ist.

4. Verbindung nach Anspruch 1, dadurch gekennzeichnet, daß sie 1-N-[2-[[5-[(Dimethylamino)-methyl]-furfuryl]-thio]-äthyl]-amino-1-(4-methylpiperidinyl)-2-nitroäthylen ist.

5. Verbindung nach Anspruch 1, dadurch gekennzeichnet, daß sie N-[2-[[5-[(Dimethylamino)-methyl]-furfuryl]-thio]-äthyl]-N'-(N-äthylpiperidinyl)-2-nitro-1.1-äthylendiamin ist.

6. Verbindung nach Anspruch 1, dadurch gekennzeichnet, daß sie 1-N-[2-[[5-[(Dimethylamino)-methyl]-furfuryl]-thio]-äthyl]-amino-[4-(2-hydroxyäthyl)-1-piperazinyl]-2-nitroäthylen ist.

7. Verbindung nach Anspruch 1, dadurch gekennzeichnet, daß sie 1-N-[2-[[5-[(Dimethylamino)-methyl]-furfuryl]-thio]-äthyl]-amino-1-(4-benzylpiperidinyl)-2-nitroäthylen ist.

8. Verbindung nach Anspruch 1, dadurch gekennzeichnet, daß sie 1-N-[2-[[5-[(Dimethylamino)-methyl]-furfuryl]-thio]-äthyl]-amino-1-(4-benzyl-1-piperidinyl)-2-nitroäthylen ist.

9. Verbindung nach Anspruch 1, dadurch gekennzeichnet, daß sie 1-N-[2-[[5-[(Dimethylamino)-methyl]-furfuryl]-thio]-äthyl]-amino-1-äthylenimino-2-nitroäthylen ist.

10. Verbindung nach Anspruch 1, dadurch gekennzeichnet, daß sie 1-N-[2-[[5-[(Dimethylamino)-methyl]-furfuryl]-thio]-äthyl]-amino-1-propylenimino-2-nitroäthylen ist.

11. Verbindung nach Anspruch 1, dadurch gekennzeichnet, daß sie N-[2-[[5-[(Dimethylamino)-methyl]-furfuryl]-thio]-äthyl]-N'-cyclohexyl-2-nitro-1.1-äthylendiamin ist.

12. Verbindung nach Anspruch 1, dadurch gekennzeichnet, daß sie N-[2-[[5-[(Dimethylamino)-methyl]-furfuryl]-thio]-äthyl]-N'-(1,4-cyclohexadienyl)-2-nitro-1.1-äthylendiamin ist.

13. Verbindung nach Anspruch 1, dadurch gekennzeichnet, daß sie 1-N-[2-[[5-[(Dimethylamino)-methyl]-furfuryl]-thio]-äthyl]-amino-1-hexamethylenimino-2-nitroäthylen ist.

14. Verbindung nach Anspruch 1, dadurch gekennzeichnet, daß sie N-[2-[[5-[(Dimethylamino)-methyl]-furfuryl]-thio]-äthyl]-N'-(N-hexamethylenimino)-amino-2-nitro-1,1-äthylendiamin ist.

15. Verbindung nach Anspruch 1, dadurch gekennzeichnet, daß sie N-[2-[[5-[(Dimethylamino)-methyl]-furfuryl]-thio]-äthyl]-N'-cycloheptyl-2-nitro-1,1-äthylendiamin ist.

16. Verbindung nach Anspruch 1, dadurch gekennzeichnet, daß sie N-[2-[[5-[(Dimethylamino)-methyl]-furfuryl]-thio]-äthyl]-N'-cyclopentyl-2-nitro-1,1-äthylendiamin ist.

17. Verbindung nach Anspruch 1, dadurch gekennzeichnet, daß sie 1-N-[2-[[5-[(Dimethylamino)-methyl]-furfuryl]-thio]-äthyl]-amino-1-pyrrolidinyl-2-nitroäthylen ist.

18. Verbindung nach Anspruch 1, dadurch gekennzeichnet, daß sie 1-N-[2-[[5-[(Dimethylamino)-methyl]-furfuryl]-thio]-äthyl]-amino-1-morpholinyl-2-nitroäthylen ist.

19. Verbindung nach Anspruch 1, dadurch gekennzeichnet, daß sie N-[2-[[5-[(Dimethylamino)-methyl]-furfuryl]-thio]-äthyl]-N'-(endonorbornyl)-2-nitro-1,1-äthylendiamin ist.

20. Verbindung nach Anspruch 1, dadurch gekennzeichnet, daß sie 1-N-[2-[[5-[(Dimethylamino)-methyl]-furfuryl]-thio]-äthyl]-N'-(norbornen-2-yl)-2-nitro-1,1-äthylendiamin ist.

21. Verbindung, dadurch gekennzeichnet, daß sie das pharmazeutisch annehmbare Salz einer Verbindung nach einem der Ansprüche 3 bis 20 ist.

22. Verfahren zum Herstellen von Verbindungen der Formel (I), wie in Anspruch 1 definiert, und deren pharmazeutisch annehmbarer Salze, dadurch gekennzeichnet, daß 2-[[5-[(Dimethylamino)-methyl]-furfuryl]-thio]-äthylmethylsulfid der folgenden Formel (III)

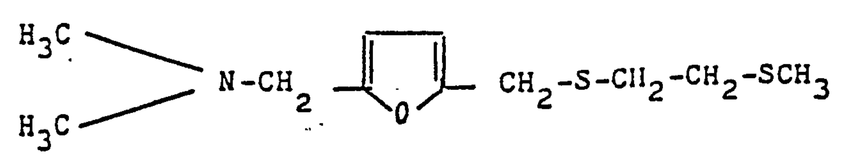

(III)

mit einer Verbindung der folgenden Formel (II)

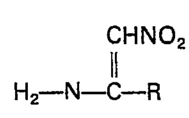

(II)

worin R wie in Anspruch 1 definiert ist, bei einer Temperatur von 80°C reagieren gelassen wird, worauf das erhaltene Produkt isoliert und gegebenenfalls in das gewünschte Salz überführt wird.

23. Verfahren nach Anspruch 22, dadurch gekennzeichnet, daß ein überschuß dere Verbindung der Formel (II) verwendet wird und die Reaktionsmischung 3 h bei 80°C gehalten wird.

24. Verfahren nach einem der Ansprüche 22 und 23, dadurch gekennzeichnet, daß die Verbindung der Formel (II) durch Umsetzen von 1,1-Bis-(methylthio)-2-nitroäthylen der folgenden Formel (IV)

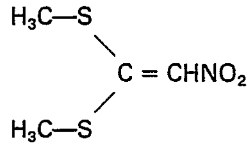

(IV)

mit einer Verbindung der folgenden Formel

RH (V)

worin R wie in Anspruch 1 definiert ist, gefolgt von Umsetzung mit Ammoniak, erhalten wird.

25. Pharmazeutisch Zusammensetzung, dadurch gekennzeichnet, daß sie als wirksamen Bestandteil einen wirksamen Anteil einer oder mehrerer Verbindungen nach den Ansprüchen 1 bis 21 an sich oder kombiniert mit anderen verträglichen Wirkstoffen und/oder Trägern, Verdünnungsmitteln, Lösungsmitteln und/oder Exzipienten, die pharmazeutisch annehmbar sind, enthält.

26. Zusammensetzung nach Anspruch 25, dadurch gekennzeichnet, daß sie für orale, Injektions-, rektale oder topische Verarbreichung geeignet ist.

27. Zusammensetzung nach Anspruch 26, dadurch gekennzeichnet, daß sie in Pillenform ist.

28. Zusammensetzung nach Anspruch 26, dadurch gekennzeichnet, daß sie in Form einer Pille mit verlängerter Wirkung ist, wobei der Wirkstoff in kleinen Anteilen Während eines Zeitraumes freigesetzt wird.

**Patentansprüche fur den Vertragsstaat: AT**

1. Verfahren zur Herstellung von Verbindungen der Formel

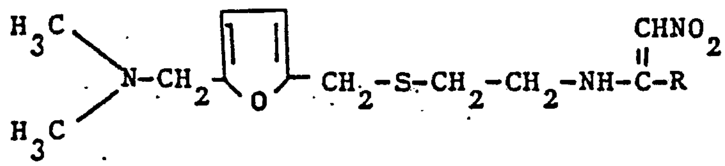

(I),

worin R eine Piperidinogruppe, 4-Methylpiperidin, (3-N-Äthylpiperidinyl)-amin, 4-(2-Hydroxyäthyl)-1-piperazin, 4-Benzylpiperidin, 4-Benzylpiperazin, Äthylenimin, Propylenimin, Cyclohexylamin, 1,4-Cyclohexydienylamin, Hexamethylenimin, (N-Hexamethylenimino)-amin, Cycloheptylamin, Cyclopentylamin, Pyrrolidin, Morpholin, Endo-2-norbornylamin, Norbornen-2-ylamin bedeutet, und deren pharmazeutisch annehmbarer Salze, dadurch gekennzeichnet, daß 2-[[5-[(Dimethylamino)-methyl]-furfuryl]-thio]-äthylmethylsulfid der Formel

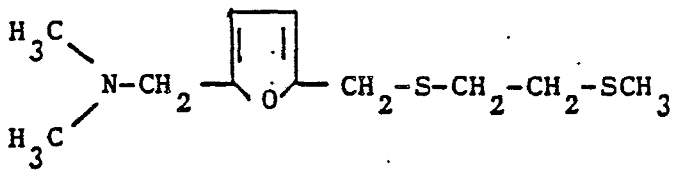

(III)

mit einer Verbindung der Formel

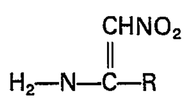

(II)

worin R wie oben definiert ist, bei einer Temperatur von 80°C umgesetzt wird, worauf das erhaltene Produkt isoliert und gegebenenfalls in das gewünschte Salz überführt wird.

2. Verfahren nach Anspruch 1, dadurch gekennzeichnet, daß ein überschuß der Verbindung der Formel (II) verwendent wird und die Reaktionsmischung 3 h bei 80°C gehalten wird.

3. Verfahren nach Anspruch 1 und 2, dadurch gekennzeichnet, daß die Verbindung der Formel (II) durch Umsetzen von 1,1-Bis-(methylthio)-2-nitroäthylen der Formel

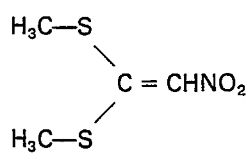

(IV)

mit einer Verbindung der Formel

RH (V)

worin R wie in Anspruch 1 definiert ist, gefolgt von der Umsetzung mit Ammoniak, erhalten wird.

**Revendications pour les Etats contractants: BE CH DE FR GB LI LU NL SE**

1. Composés de formula (I)

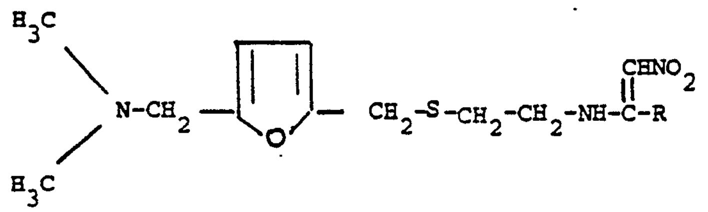

(I)

dans laquelle R est un groupe pipéridine, 4-méthylpipéridine, (3-N-éthyl-pipéridinyl)amine, 4-(2-hydroxy-éthyl)-1-pipérazine, 4-benzyl-pipéridine, 4-benzyl-pipéridine, éthylènimine, propylèneimine, cyclohexylamine, 1,4-cyclohexadiénylamine, hexaméthylèneimine, (N-hexaméthylèneimine) amine, cycloheptylamine, cyclopentyl-amine, pyrrolidine, morpholine, endo-2-norbornylamine, norbornèn-2-yl-amine et leurs sels pharmaceutiquement acceptables.

2. Composé selon la revendication 1, caractérisé en ce que le sel pharmaceutiquement acceptable est le sel obtenu avec les acides chlorhydrique, bromhydrique, iodhydrique, phosphorique, sulfurique, maléique, acétique, citrique, oxalique, succinique, benzoïque, tartrique, fumarique, mandélique, ascorbique, méthylsulfonique ou éthylsulfonique.

3. Composé selon la revendication 1, caractérisé en ce que c'est le 1-N-[2-[5-[(diméthyl-amino)méthyl]furfuryl]thio]éthyl]-amino-1-pipéridinyl-2-nitro éthylène.

4. Composé selon la revendication 1, caractérisé en ce que c'est le 1-N-[2-[5-[(diméthyl-amino)méthyl]furfuryl]thio]éthyl]-amino-1-(4-methyl-pipéridinyl)-2-nitro éthylène.

5. Composé selon la revendication 1, caractérisé en ce que c'est le 1-N-[2-[5-[(diméthyl-amino)méthyl]furfuryl]thio]éthyl]-N'-(N-éthylpipéridinyl)-2-nitro-1,1-éthylènediamine.

6. Composé selon la revendication 1, caractérisé en ce que c'est le 1-N-[2-[5-[(diméthyl-amino)méthyl]furfuryl]thio]éthyl]-amine-[4-(2-hydroxyéthyl)-1-pipérazinyl]-2-nitro éthylène.

7. Composé selon la revendication 1, caractérisé en ce que c'est le 1-N-[2-[5-[(diméthyl-amino)méthyl]furfuryl]thio]éthyl]-amine-1-(4-benzyl-pipéradinyl]-2-nitro éthylène.

8. Composé selon la revendication 1, caractérisé en ce que c'est le 1-N-[2-[5-[(diméthyl-amino)méthyl]furfuryl]thio]éthyl]-amine-1-(4-benzyl-1-pipérazinyl]-2-nitro éthylène.

9. Composé selon la revendication 1, caractérisé en ce que c'est le 1-N-[2-[5-[(diméthyl-amino)méthyl]furfuryl]thio]éthyl]-amine-1-éthylèneimino-2-nitro éthylène.

10. Composé selon la revendication 1, caractérisé en ce que c'est le 1-N-[2-[5-[(diméthyl-amino)méthyl]furfuryl]thio]éthyl]-amine-1-propylèneimino-2-nitro éthylène.

11. Composé selon la revendication 1, caractérisé en ce que c'est le N-[2-[5-[(diméthyl-amino)méthyl]furfuryl]thio]éthyl]-N'-cyclohexyl-2-nitro-1-éthylènediamine.

12. Composé selon la revendication 1, caractérisé en ce que c'est le N-[2-[5-[(diméthyl-amino)méthyl]furfuryl]thio]éthyl]-N'-(1,4-cyclohexadiényl)-2-nitro-1-éthylènediamine.

13. Composé selon la revendication 1, caractérisé en ce que c'est le N-[2-[5-[(diméthyl-amino)méthyl]furfuryl]thio]éthyl]-amine-1-hexaméthylèneimino-2-nitroéthylène.

14. Composé selon la revendication 1, caractérisé en ce que c'est le N-[2-[5-[(diméthyl-amino)méthyl]furfuryl]thio]éthyl]-N'-(N-hexaméthylèneimino)amino-2-nitro-1,1-éthylènediamine.

15. Composé selon la revendication 1, caractérisé en ce que c'est le N-[2-[5-[(diméthyl-amino)méthyl]furfuryl]thio]éthyl]-N'-cycloheptyl-2-nitro-1,1-éthylènediamine.

16. Composé selon la revendication 1, caractérisé en ce que c'est le N-[2-[5-[(diméthyl-amino)méthyl]furfuryl]thio]éthyl]-N'-cyclopentyl-2-nitro-1,1-éthylènediamine.

17. Composé selon la revendication 1, caractérisé en ce que c'est le 1-N-[2-[5-[(diméthyl-amino)méthyl]furfuryl]thio]éthyl]-amine-1-pyrrolidinyl-2-nitro éthylène.

18. Composé selon la revendication 1, caractérisé en ce que c'est le 1-N-[2-[5-[(diméthyl-amino)méthyl]furfuryl]thio]éthyl]-amine-1-morpholinyl-2-nitro éthylène.

19. Composé selon la revendication 1, caractérisé en ce que c'est le N-[2-[5-[(diméthyl-amino)méthyl]furfuryl]thio]éthyl]-N'-(endonorbornyl)-2-nitro-1,1- éthylènediamine.

20. Composé selon la revendication 1, caractérisé en ce que c'est le N-[2-[5-[(diméthyl-amino)méthyl]furfuryl]thio]éthyl]-N'-(norbornèn-2-yl)-2-nitro-1,1-éthylènediamine.

21. Composé caractérisé en ce qu'il est constitué par le sel pharmaceutiquement acceptable d'un composé selon l'une des revendications 3 à 20.

22. Procédé de préparation des composés de formule (I), tels que définis dans la revendication 1, et leurs sels pharmaceutiquement acceptables, caractérisé en ce que l'on fait réagir le 2-[[5-[(diméthyl-amino)méthyl]furfuryl]thio]éthylméthylsulfure de formule (III),

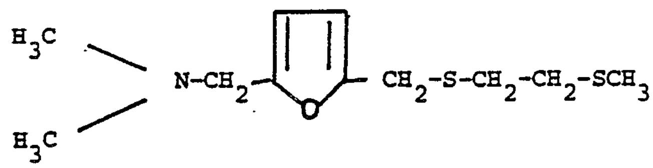
(III)

avec un composé répondant à la formule ci-dessous

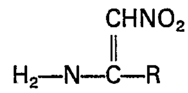
(II)

dans laquelle R a la même signification que dans la revendication 1, à une température de 80°C, après quoi le produit obtenu est isolé et éventuellement, converti en son sel souhaité.

23. Procédé selon la revendication 22, caractérisé en ce que l'on utilise le composé de formule (II) en excès et que le mélange réactionnel est maintenu à 80°C pendant trois heures.

24. Procédé selon l'une des revendications 22 et 23, caractérisé en ce que le composé de formule (II) est obtenu par la réaction entre la 1,1-bis-(méthylthio)-2-éthylène, de formule IV ci-dessous

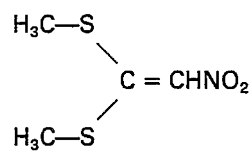
(IV)

et un composé de formule (V) ci-dessous

RH (V)

dans laquelle R a la même signification que dans la revendication 1, suivie par une réaction avec de l'ammoniac.

25. Composition pharmaceutique caractérisée en ce qu'elle contient, comme principe actif, une quantité efficace d'un ou de plusieurs des composés selon les revendications 1 à 21, tel quel ou combiné avec d'autres principesactifs compatibles et/ou des véhicules, diluants, solvants et/ou excipients pharmaceutiquement acceptables.

26. Composition selon la revendication 25, caractérisé en ce qu'elle est adaptée à l'administration par voie orale, par injection, par voie rectale ou locale.

27. Composition selon la revendication 26, caractérisée en ce qu'elle est sous forme de pilule.

28. Composition selon la revendication 26, caractérisé en ce qu'elle est sous la forme d'une pilule à action prolongée, le principe actif étant libéré en petites quantités pendant une certaine durée.

**Revendications pour l'Etat contractant: AT**

1. Procédé de préparation de composés de formule

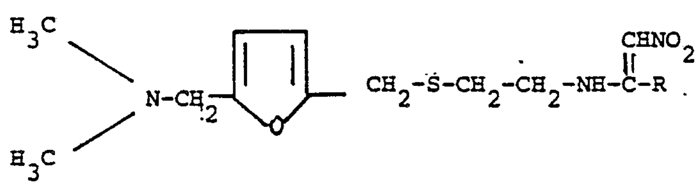
(I)

dans laquelle R représente un groupe pipéridine, 4-méthylpiperidine, (3-N-éthyl-pipéridinyl)amine, 4-(2-hydroxyéthyl)-1-pipérazine, 4-benzyl-pipéridine, 4-benzyl-pipérazine, éthylèneimine, propylèneimine, cyclohexylamine, 1,4-cyclohexadiènylamine, hexaméthylèneimine, (N-hexaméthylèneimino)amine, cycloheptylamine, cyclopentylamine, pyrrolidine, morpholine, endo-2-norbornylamine, norbornèn-2-yl-amine, et leurs sels pharmaceutiquement acceptables, procédé caractérisé en ce que l'on fait réagir le 2-[[5-[(diméthylamino)méthyl]furfuryl]thio]éthylméthylsulfure de formule

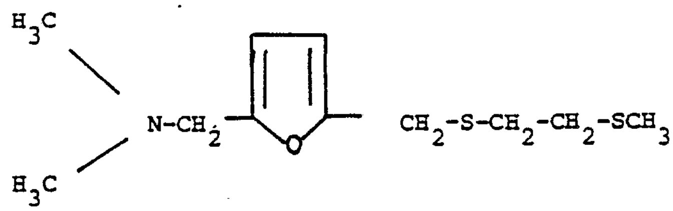

(III)

avec un composé de formule

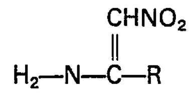

(II)

dans laquelle R a la même signification que ci-dessus, à une température de 80°C, après quoi le produit obtenu est isolé et, éventuellement, converti en son sel dérivé.

2. Procédé selon la revendication 1, caractérisé en ce que l'on utilise un excès en composé de formule (II) et que le mélange réactionnel est maintenu à 80°C pendant trois heures.

3. Procédé selon les revendications 1 et 2, caractérisé en ce que le composé de formule (II) est obtenu par réaction entre le 1,1-bis-(méthylthio)-2-nitro-éthylène de formule

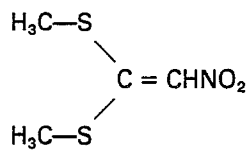

(IV)

et un composé de formule

$$RH \qquad (V)$$

dans laquelle R a la même signification que dans la revendication 1, suivie d'une réaction avec l'ammoniac.